# EUROPEAN PATENT APPLICATION

(11) **EP 4 393 497 A2**
(43) Date of publication of application: **03.07.2024**
(21) Application number: 24167332.6
(22) Date of filing: 18.08.2022
(51) Int. Cl.: A61K 33/30

(54) **METHOD FOR PREVENTING ENTRY AND REPLICATION OF ENVELOPED VIRUSES**

(30) Priority: 22.08.2021 US 202163235772 P; 11.09.2021 US 202117472604; 27.12.2021 US 202163294067 P; 10.02.2022 US 202263308782 P; 08.08.2022 US 202263396040 P
(62) Divisional of application: 22861909.4
(71) Applicant: Viron, Inc., Boston, MA 02108 (US)
(72) Inventor: JENA, Bhanu Pratap, Bloomfield Hills, 48302 (US)
(74) Representative: Modiano, Gabriella Diana

(57) **Abstract**

The invention provides the use of a formulation containing cyclodextrin, quercetin and zinc at appropriate concentrations to mitigate infections by enveloped viruses. While the different forms of cyclodextrin prevent the entry of coated viruses into host cells by extracting and sequestering cholesterol molecules at the virus envelope and at the host cell plasma membrane, the ionophore quercetin enables cellular entry of zinc, thereby inhibiting viral replication by altering polymerase activity in the host cell.

## Description

### APPLICATION CROSS-REFERENCE

The instant Application claims priority to U.S. Prov. Pat. App. No. 63/396,040, filed on 08 August 2022, U.S. Prov. Pat. App. No. 63/308,782, filed 10 February 2022, U.S. Prov. Pat. App. No. 63/235,772, filed on 22 August 2021, and U.S. Prov. Pat. App. No. 63/294,067 filed 27 December 2021, and is a continuation-in-part application of U.S. Pat. App. No. 17/472,604, filed 11 September 2021, which application claims priority to U.S. Prov. Pat. App. No. 63/235,772, filed on 22 August 2021, and which is a continuation-in-part of U.S. Pat. App. No. 17/207,250, filed 19 March 2021, which claims priority to U.S. Prov. Pat. App. No. 63/029,458 filed 23 May 2020 and U.S. Prov. Pat. App. No. 63/019,312 filed 02 May 2020, the entireties of which are incorporated by reference herein in their entireties.

### BACKGROUND

### TECHNICAL FIELD

Certain embodiments of the present invention relate to the prevention of enveloped viral entry into cells. Still other embodiments prevent the replication of enveloped viruses should cell entry occur.

### DISCUSSION OF ART

Viruses enter hosts via the epithelium. The cell plasma membrane of skin and lung epithelia is the first line of defense and when breached serves as the portal for viral entry into hosts. Studies in the past two decades report the various cell membrane binding and entry mechanisms utilized by viruses to infect. Regardless of the different mechanisms involved in viral entry into a host cell, the initiating critical process is binding of the virus particle to a host cell plasma membrane. Without the binding of a virus to the cell plasma membrane, there would be no viral entry into the host.

It is established that viral binding to a host cell membrane is subject to the presence of docking sites or receptors which, in turn, are regulated by membrane lipid composition and distribution. An example is the establishment of domains called rafts. A study of cellular membrane biogenesis demonstrated that depletion of membrane cholesterol, impacts both the chemistry and distribution of plasma membrane proteins and lipids, impacting cell function. Cells exposed to an increasing concentration of methyl-beta-cyclodextrin (M-βCD) to deplete cholesterol from the cell plasma membrane demonstrate loss in the uptake of phosphotidyl serine by the cell plasma membrane, while the uptake of phosphatidylethanolamine remain unchanged. Similarly, the loss of cholesterol from the cell plasma membrane resulted in the depletion of membrane fusion proteins, such as syntaxin and SNAP25 from the plasma membrane, suggesting altered membrane fusogenicity. Therefore, changes to the chemistry of the epithelial cell plasma membrane via depletion of sterols/cholesterol by cyclodextrins ("CDs") could dictate both the binding of the virus at the cell plasma membrane, and influence both the efficacy and potency of its entry into the host cell.

Further studies demonstrated that depletion of plasma membrane cholesterol in host cells using M-βCD, significantly reduces entry of the pseudorabies and vaccinia virus into cells. Additional similar studies likewise demonstrated that HIV infectivity is critically dependent on cholesterol presence at the cell plasma membrane.

Multiple studies suggest that cholesterol rich microdomains, called lipid 'rafts' facilitate the cellular entry or infection of HIV, its assembly, and its release from infected cells. Lipid rafts are domains within the lipid bilayer, usually localized to the external leaf of the bilayer, present in many cell types, and implicated in a variety of sorting and signaling processes. These lipid rafts tend to be enriched in cholesterol and sphingolipids. Additional studies further report that plasma membrane cholesterol is also required for a wide range of both bacterial and yeast infections. Furthermore, a high-cholesterol diet impairs pulmonary host defense against gram-negative bacteria and *Mycobacterium* tuberculosis.

Cyclodextrins are a family of cyclic oligosaccharides constituted of a macrocyclic ring of glucose subunits joined by α-1,4 glycosidic bonds. CDs are used for improving the water-solubility and bioavailability of a wide range of drugs. The U.S. Food and Drug Administration (FDA) has approved the use of cyclodextrins since 2001. Cyclodextrins were first employed in the food industry in the 1970s, and since then they have been used as food additives for carrying food-related lipophiles such as vitamins, aromas and colorants.

βCD has also been used as a cholesterol-reducing agent in food of animal origin such as milk and egg. The first pharmaceutical patent related to CDs and pharmaceutical applicability as complexing agents is dated 1953. Currently, CDs are employed in pharmaceutical products primarily to increase water solubility of poorly soluble drug formulations and to enhance drug bioavailability. Pharmaceutical products containing CDs comprise nasal spray, oral solutions, solid dosage forms, ocular and dermal formulations, suppositories, and parenteral solutions. Currently, more than 40 pharmaceutical products containing CDs are available on the market worldwide, and many of them utilize βCD and its derivatives having higher water solubility, such as HPβCD, MβCD, and SBEβCD. Most of the βCD are also approved by the European Medical Agency for all human administration pathways. CDs are used for example in tablets, aqueous parenteral solutions, nasal sprays, and eye drop solutions. Examples of the use of cyclodextrins in medicines on the European market are: β-CD in cetirizine tablets and cisapride suppositories; γ-CD in minoxidil solution. Examples of the use of β-cyclodextrin derivatives can include: SBE-β-CD in the intravenous antimycotic voriconazole; HP-β-CD in the antifungal itraconazole intravenous and oral solutions; and, RM-β-CD in a nasal spray for hormone replacement therapy by 17β-estradiol. In Germany and Japan there are infusion products on the market, containing alprostadil (prostaglandin E1, PGE1) with α-CD. Cyclodextrins are currently not included in the European Commission Guideline on excipients in the label and package leaflet of medicinal products for human use.

Despite all the advancements above cataloged, there remains a need to treat infection with or exposure to an enveloped virus in a therapeutic or prophylactic fashion. In particular, there remains a need to stabilize or reduce the amount of virus present in a human or other animal.

### BRIEF SUMMARY OF THE INVENTION

It is an object of the invention to provide cyclodextrins as a drug in different particle sizes in phosphate buffered solutions. CDs may be at concentrations between one (1) and ten (10) percent.

It is an object of the invention to deliver solutions of CDs via aerosol spray or nebulizer. A particular cyclodextrin may be hydroxypropyl-beta-cyclodextrin (HPβCD) in an aqueous phosphate-buffered saline solution. Such solution may be at a pH of 5-7.5. Further embodiments may contain 0.01% benzylkonium chloride. In additional embodiments the cyclodextrin may be at least one selected from the group of: αCD, βCD, yCD, MβCD, and SBEβCD.

It is an object of the invention to administer solutions, such as those described herein, prophylactically to reduce the entry and/or replication of respiratory viruses.

In certain embodiments 1-20 µg mL⁻¹ quercetin is administered as part of the mixture.

In still other embodiments, solubilized zinc is administered as part of the formulation.

In some embodiments the solubilized zinc may administered at a concentration of 2-10 µg mL⁻¹.

In still other embodiments, the solution is provided at pH 2.5 and the concentration of benzylkonium is at 0.01%.

In another embodiment, the solution is provided at pH 7.5 with a benzylkonium concentration of 0.01%.

In still other embodiments, the solution may be applied topically to the skin either as a spray or as a wipe. Still other embodiments include application via a cellulose mask.

### DETAILED DESCRIPTION OF THE INVENTION

CDs such as αCD, βCD, and γCD are hydrophilic in aqueous solutions, however they tend to self-assemble and form complexes. To overcome this limitation, soluble βCD derivatives such as 2-hydroxypropyl-βCD (HPβCD) and sulfobutyl ether βCD sodium salt (SBEβCD), are preferred for use in aqueous pharmaceutical formulations. Inorganic acids such as phosphoric and citric acid induce CD solubilization.

Zinc is an essential trace element supporting growth, development and immune health. Zinc is also known to protect against viruses by inhibiting RNA binding, RNA synthesis, viral polyprotein cleavage, viral replication, and viral protease enzyme inactivation. However, in order to protect against viral infection, zinc needs to enter a host cell. Quercetin, a naturally occurring plant-based over-the-counter zinc ionophore, enables the cellular entry of zinc into host cells. Furthermore, quercetin has shown therapeutic effects against influenza virus. Additionally, *in silico* modelling of the interactions between the SARS-CoV-2 viral spike protein and the epithelial cell angiotensin converting enzyme-2 (ACE2) protein, has identified quercetin from a database of 8,000 small molecule candidates of known drugs, metabolites, and natural products, as one of the top 5 most potent compounds for binding to the interface site, and disrupt initiation of viral infection.

Formulations of the present invention utilize CDs at FDA approved concentrations (e.g., between one (1) and ten (10) percent weight per volume; 1-10% w v⁻¹) in combination with quercetin and zinc in pH buffered solutions to retain both high solubility and sterility. Modes of administration can include aerosol spray, nebulization; and, topical application on the surface of a body using a water-based solution adsorbed to paper, cellulose or fabric. The topical application on body surfaces includes specific application to the face and neck, to mitigate envelope virus (*e.g.,* COVID-19 virus (SARS-CoV-2), influenza, and HIV), bacterial and fungal infections.

The invention provides the use of a formulation containing cyclodextrins, quercetin and zinc, at appropriate concentrations to mitigate infections by enveloped viruses, such as COVID-19 virus (SARS-CoV-2), influenza, and HIV. This formulation may be adapted for both topical use or respiratory system introduction to a subject by dispersal utilizing known methods such as measured nasal sprays, nebulizers, inhalers, wet wipes and medicated masks.

In some embodiments the formulation is introduced in the form of an aqueous phosphate buffered saline pH 7.5 solution, containing 0.01% benzylkonium chloride as preservative and different concentrations of the active ingredients: 1-5% cyclodextrin; the flavonoid quercetin at a concentration of 8 µg mL⁻¹; and, 1 mg mL⁻¹ zinc chloride. The aqueous solution may be in some embodiments citrate buffered at pH 2.5. The low pH serves as a preservative and a solvent for CDs.

Without subscription or limitation to a specific theory, it is believed that the different forms of cyclodextrin molecules prevent the entry of enveloped viruses into host cells by extracting and sequestering cholesterol molecules at the virus coat/envelope and at the host cell plasma membrane. The quercetin in the formulation enables cellular entry of zinc; which, in turn, inhibits viral replication by altering polymerase activity in the host cell.

More specifically, it is believed CDs will allow the extraction of cholesterol molecules from enveloped virus membranes and the host cell membrane, altering their respective lipid and protein composition and distribution, preventing virus entry into host cells. The quercetin, acting as a zinc ionophore, enables the cellular entry of zinc. The zinc functions to protect host cells against the envelope virus by inhibiting RNA binding, RNA synthesis, viral polyprotein cleavage, viral replication, and viral protease enzyme inactivation, among others. Thus, both viral entry and replication are prevented thereby decreasing the viral particle load present in or on a subject. Therefore, embodiments of the invention may be used prophylactically to prevent infection, or, may be used therapeutically to alleviate the associated effects of viral exposure and/or infection.

Certain embodiments provide an aerosol spray and nebulization of the combined cyclodextrin, quercetin and zinc as a aqueous phosphate buffered saline pH 7.5 solution containing 0.01% benzylkonium chloride as preservative. The embodiment is administered as a prophylactic to protect the airways, including the lungs, from infections with enveloped viruses. In some embodiments different particle sizes may be combined (e.g., a nebulizer treatment supplemented with an inhaler).

Similarly, topical application of the combined cyclodextrin, quercetin and zinc in aqueous phosphate buffered saline pH 7.5 solution containing 0.01% benzylkonium chloride as preservative, will be used to protect body surface (skin) from infections with enveloped viruses. a particular embodiment, the formulation is adapted for use and introduction to a subject by dispersal, utilizing any known method of measured nasal sprays or inhalers. The formulation is introduced in form of an aqueous phosphate buffered saline pH 5-7.5 solution, containing 0.01% benzylkonium chloride as preservative and different concentrations of the active ingredients: 1-5% cyclodextrin; and the flavonoid quercetin, a naturally occurring zinc ionophore at a concentration of 8-24 µg ml⁻¹. Depending on requirement, pH 2.5 citrate buffered aqueous may also be used, where the low pH serves as a preservative and a solvent for cyclodextrin.

In another embodiment, administration of cyclodextrin alone is followed by quercetin and zinc administration (alone or combined), in a water based soluble formulation that prevents both viral entry and replication in host cells, such as the nasal epithelial cells and that of the lung epithelia. The formulation may be administered as an aerosol spray or nebulization. A particular embodiment contemplates an aqueous phosphate buffered saline pH 7.5 solution containing 0.01% benzylkonium chloride as preservative, will be used to protect the airways including lungs from all coat virus infections.

In additional embodiments the above formulations may be topically applied; for example, via oils, creams, emulsions and the like the formulations of which are known to those skilled in the art. Additional embodiments include application of the above solutions to both sides of cellulose masks. In such medicated masks, any airborne droplets containing the virus will be neutralized on contact with the medicated mask.

Suitable alterations to the above are readily apparent to those of skill in the art and naturally are encompassed and expressly contemplated. For example, normal manufacturing tolerances may induce variances from the above presented formulations without departing from the broader scope of this invention. Any of the compounds used in embodiments of the invention may be obtained from supply companies as commodities of various grades and qualities needing more or less refinement, as known in the art, for usage in the above-described applications.

The effective dose and method of administration of a particular embodiment of the instant invention may vary based on the individual patient and stage of any present diseases (e.g., influenza, covid, HIV, other co-morbidities), as well as other factors known to those of skill in the art. Dosages of the above described embodiments result in a decrease in viral particle load through the above described mechanisms of action. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions that exhibit large therapeutic indices are preferred. The data obtained from cell culture assays and animal studies is used in formulating a range of dosage for human use. The dosage of such compounds lies preferably within a range of circulating concentrations that include the ED50 with little or no toxicity. The dosage varies within this range depending upon the dosage form employed, sensitivity of the patient, and the route of administration.

The exact dosage may be chosen by an individual physician in view of a patient to be treated. Dosage and administration are adjusted to provide sufficient levels of embodiments of the instant invention to maintain the desired effect (e.g., elimination or reduction of enveloped virus particles or activity in a host). Additional factors that may be taken into account include the severity of any disease state, age, weight, and gender of the patient; diet, time and frequency of the administration, drug combination(s), reaction sensitivities, and tolerance/response to therapy.

Short acting pharmaceutical compositions are administered daily whereas long acting pharmaceutical compositions are administered every 2, 3 to 4 days, every week, or once every two weeks or more. Depending on half-life and clearance rate of the particular formulation, the pharmaceutical compositions of the instant invention may be administered once, twice, three, four, five, six, seven, eight, nine, ten or more times per day.

Normal dosage amounts for active ingredients may vary from approximately 1 to 100,000 micrograms, up to a total dose of about 10 grams, depending upon the route of administration. Desirable dosages include 250 µg, 500 µg, 1 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 1 g, 1.1 g, 1.2 g, 1.3 g, 1.4 g, 1.5 g, 1.6 g, 1.7 g, 1.8 g, 1.9 g, 2 g, 3 g, 4 g, 5 g, 6 g, 7 g, 8 g, 9 g, and 10 g.

More specifically, the dosage of the active ingredients described herein are those that provides sufficient quantity to attain a desirable effect, including those above-described effects. Accordingly, the dose of the active ingredients preferably produces a tissue or blood concentration of both about 1 to 800 µM. Preferable doses produces a tissue or blood concentration of greater than about 10 µM to about 500 µM. Preferable doses are, for example, the amount of active ingredients required to achieve a tissue or blood concentration or both of 10 µM, 15 µM, 20 µM, 25 µM, 30 µM, 35 µM, 40 µM, 45 µM, 50 µM, 55 µM, 60 µM, 65 µM, 70 µM, 75 µM, 80 µM, 85 µM, 90 µM, 95 µM, 100 µM, 110 µM, 120 µM, 130 µM, 140 µM, 150 µM, 160 µM, 170 µM, 180 µM, 190 µM, 200 µM, 220 µM, 240 µM, 250 µM, 260 µM, 280 µM, 300 µM, 320 µM, 340 µM, 360 µM, 380 µM, 400 µM, 420 µM, 440 µM, 460 µM, 480 µM, and 500 µM. Although doses that produce a tissue concentration greater than 800 µM are not necessarily preferred, they are envisioned and can be used with some embodiments of the present invention. A constant infusion of embodiments of the invention can be provided so as to maintain a stable concentration of the therapeutic agents.

Finally, the written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

The pharmacologically active compounds of this invention can be processed in accordance with conventional methods of galenic pharmacy to produce medicinal agents for administration to patients (e.g., mammals including humans).

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property.

Since certain changes may be made in the above-described invention, without departing from the spirit and scope of the invention herein involved, it is intended that all of the subject matter of the above description shall be interpreted merely as examples illustrating the inventive concept herein and shall not be construed as limiting the invention.

The present invention is further defined by the following items:
1. A method for the reduction of envelope viral particle number comprising: administering to
   a subject an aqueous solution comprised at least of: cyclodextrin,
   quercetin, and zinc resulting in a decrease of envelope viral particles.
2. The method of item 1 further comprising: benzylkonium chloride.
3. The method of item 2 wherein the benzylkonium chloride is at 0.01%
4. The method of item 1 wherein the quercetin is at a concentration of 1-20 µg mL⁻¹.
5. The method of item 1 wherein the zinc is at a concentration of 2-10 µg mL⁻¹.
6. The method of item 1 wherein the solution is at pH 2.5 and the concentration of benzykonium chloride is at 0.01%.
7. The method of item 6 further comprising: elevating the pH of the solution from 2.5 to 7.5 prior to administration.
8. The method of item 1 wherein the administration is accomplished via a nebulizer.
9. The method of item 1 wherein the administration is accomplished via an inhaler.
10. The method of item 1 wherein the administration is accomplished via a spray.
11. The method of item 1 wherein the administration is accomplished by placing a mask soaked in the aqueous solution on the subject.
12. A method for the reduction of envelope viral particles comprising:
   administering to a subject a first aqueous solution of cyclodextrin; administering to a subject a second aqueous solution of quercetin and zinc.
13. The method of item 12 wherein the first and second aqueous solutions are administered from at least one selected from the group: an inhaler, a nebulizer, and a nasal spray.
14. The method of item 12 wherein the first and second aqueous solutions contain 0.01% benzylkonium chloride.
15. The method of item 12 wherein the first and second aqueous solutions are at pH 7.5
16. The method of item 12 wherein the first and second aqueous solutions are at an initial pH of 2.5 that is raised to pH 7.5 prior to administration.

## Claims

1. A method for the reduction of envelope viral particles comprising:
administering to a subject a first aqueous solution of cyclodextrin; administering to a subject a second aqueous solution of quercetin and zinc.

2. The method of claim 1 wherein the first and second aqueous solutions are administered from at least one selected from the group: an inhaler, a nebulizer, and a nasal spray.

3. The method of claim 1 wherein the first and second aqueous solutions contain 0.01% benzalkonium chloride.

4. The method of claim 1 wherein the first and second aqueous solutions are at pH 7.5

5. The method of claim 1 wherein the first and second aqueous solutions are at an initial pH of 2.5 that is raised to pH 7.5 prior to administration.
